# EUROPÄISCHE PATENTANMELDUNG

(11) **EP 1 612 276 A1**
(43) Veröffentlichungstag der Anmeldung: **04.01.2006**
(21) Anmeldenummer: 05013197.8
(22) Anmeldetag: 18.06.2005
(51) Int. Cl.: C12Q 1/18, C12Q 1/48

(54) **Verfahren zum Identifizieren von fungizid wirksamen Verbindungen basierend auf Thymidylat-Kinasen**

(30) Priorität: 01.07.2004 DE 102004031901; 07.07.2004 DE 102004032872
(71) Anmelder: Bayer CropScience AG, 40789 Monheim (DE)
(72) Erfinder: Nennstiel, Dirk, Dr., 69009 Lyon (FR); Vollenbroich, Verena, Dr., 45478 Mülheim (DE); Schreier, Peter, Prof. Dr., 50674 Köln (DE)

(57) **Zusammenfassung**

Die Erfindung bezieht sich auf ein Verfahren zum Identifizieren von Fungiziden, die Verwendung von Thymidylat-Kinase zum Identifizieren von Fungiziden, die Verwendung von Inhibitoren der Thymidylat-Kinase als Fungizide und Verfahren zum Bekämpfen pflanzenpathogener Pilze.

SEQUENZPROTOKOLL

<110>  Bayer CropScience AG

<120>  Verfahren zum Identifizieren von fungizid wirksamen Verbindungen
       basierend auf Thymidylat-Kinasen

<130>  BCS033066-EP

<140>  EP05013197.8
<141>  2005-06-18

<150>  DE102004031901.4
<151>  2004-07-01

<150>  DE102004032872.2
<151>  2004-07-07

<160>  8

<170>  PatentIn version 3.3

<210>  1
<211>  240
<212>  PRT
<213>  Magnaporthe grisea

<400>  1

Met Thr Ser Thr Ser Gly Glu Thr Thr Ala Val Arg Gly Ala Leu Ile
1               5                   10                  15

Val Leu Glu Gly Leu Asp Arg Ser Gly Lys Thr Thr Gln Val Gln Leu
            20                  25                  30

Leu Glu Lys Arg Phe Leu Asp Leu Gly Arg Asn Val Lys Val Met Arg
        35                  40                  45

Phe Pro Asp Arg Thr Thr Pro Ile Gly Gln Met Ile Asp Gly Tyr Leu
    50                  55                  60

Lys Ser Gln Val Asp Met Asp Asp His Val Ile His Leu Leu Phe Ser
65                  70                  75                  80

Ala Asn Arg Trp Glu Ala Ala Lys Gln Ile Thr Asp Leu Leu Ser Ala
                85                  90                  95

Gly Thr Thr Val Leu Cys Asp Arg Tyr Tyr Tyr Ser Gly Met Val Tyr
            100                 105                 110

Ser Ala Ala Lys Gln Asn Pro Ala Leu Pro Leu Ser Trp Ala Arg Ala
        115                 120                 125

Pro Glu Val Gly Leu Pro Arg Pro Asp Leu Val Leu Phe Leu Asp Leu
    130                 135                 140

Thr Glu Glu Gln Ala Lys Ala Arg Gly Gly Trp Gly Asp Glu Leu Tyr
145                 150                 155                 160

Glu Arg Ala Glu Met Gln Arg Arg Val Arg Glu Leu Phe Trp Gly Leu
                165                 170                 175

Ser Met Gly Arg Ile Glu Ala Gly Gly Gly Ser Gln Arg Glu Glu Arg
            180                 185                 190

Ala Arg Gln Glu Glu Glu Asp Leu His Val Val Asp Ala Ser Pro Ser
        195                 200                 205

Val Glu Ala Val Cys Glu Glu Ile Trp Lys Val Val Glu Pro Arg Val
    210                 215                 220

Asp Ala Val Gly Arg Gly Glu Val Gly Lys Val Val Arg Arg Val Ser
225                 230                 235                 240

<210>   2
<211>   240
<212>   PRT
<213>   Candida albicans

<400>   2

Met Thr Ser Thr Ser Gly Glu Thr Thr Ala Val Arg Gly Ala Leu Ile
1               5                   10                  15

Val Leu Glu Gly Leu Asp Arg Ser Gly Lys Thr Thr Gln Val Gln Leu
            20                  25                  30

Leu Glu Lys Arg Phe Leu Asp Leu Gly Arg Asn Val Lys Val Met Arg
        35                  40                  45

Phe Pro Asp Arg Thr Thr Pro Ile Gly Gln Met Ile Asp Gly Tyr Leu
    50                  55                  60

Lys Ser Gln Val Asp Met Asp Asp His Val Ile His Leu Leu Phe Ser
65                  70                  75                  80

Ala Asn Arg Trp Glu Ala Ala Lys Gln Ile Thr Asp Leu Leu Ser Ala
                85                  90                  95

Gly Thr Thr Val Leu Cys Asp Arg Tyr Tyr Tyr Ser Gly Met Val Tyr
            100                 105                 110

Ser Ala Ala Lys Gln Asn Pro Ala Leu Pro Leu Ser Trp Ala Arg Ala
        115                 120                 125

Pro Glu Val Gly Leu Pro Arg Pro Asp Leu Val Leu Phe Leu Asp Leu
    130                 135                 140

Thr Glu Glu Gln Ala Lys Ala Arg Gly Gly Trp Gly Asp Glu Leu Tyr
145                 150                 155                 160

Glu Arg Ala Glu Met Gln Arg Arg Val Arg Glu Leu Phe Trp Gly Leu
                165                 170                 175

Ser Met Gly Arg Ile Glu Ala Gly Gly Gly Ser Gln Arg Glu Glu Arg
            180                 185                 190

Ala Arg Gln Glu Glu Glu Asp Leu His Val Val Asp Ala Ser Pro Ser
        195                 200                 205

Val Glu Ala Val Cys Glu Glu Ile Trp Lys Val Val Glu Pro Arg Val
    210                 215                 220

Asp Ala Val Gly Arg Gly Glu Val Gly Lys Val Val Arg Arg Val Ser
225                 230                 235                 240

<210>  3
<211>  309
<212>  PRT
<213>  Neurospora crassa

<400>  3

Met Ala Ser Thr Thr Thr Asp Pro Thr Pro Ala Thr Gln Tyr Gly Gly
1               5                   10                  15

Asn Asn Asn Pro Ile Ala Arg Gly Ala Leu Ile Val Ile Glu Gly Leu
            20                  25                  30

Asp Arg Ser Gly Lys Thr Thr Gln Val Lys Leu Leu Glu Asp Arg Phe
        35                  40                  45

Leu Lys Ala Gly Lys Lys Val Lys Thr Met Arg Phe Pro Asp Arg Thr
    50                  55                  60

Thr Pro Ile Gly Gln Met Ile Asp Ser Tyr Leu Lys Ser Gln Ala Gln
65                  70                  75                  80

Met Asp Asp His Val Ile His Leu Leu Phe Ser Ala Asn Arg Trp Glu
                85                  90                  95

Ala Val Lys Lys Ile Thr Ser Glu Leu Ser Gln Gly Thr Thr Leu Ile
            100                 105                 110

Leu Asp Arg Tyr Tyr His Ser Gly Ile Val Tyr Ser Ala Ala Lys Gln
        115                 120                 125

Asn Pro Ser Leu Ser Leu Ser Trp Ala Arg Ala Pro Glu Ile Gly Leu
    130                 135                 140

Pro Arg Pro Asp Leu Val Leu Phe Leu Asp Leu Asp Glu Glu Gln Ala
145                 150                 155                 160

Arg Leu Arg Gly Gly Trp Gly His Glu Leu Tyr Glu Lys Ala Glu Met

                165                 170                 175

Gln Arg Arg Val Arg Glu Leu Phe Trp Gly Leu Ser Leu Gly Arg Val
            180                 185                 190

Gly Thr Val Thr Val Pro Val Gly Gly Val Ser Ala Leu Gly Gly Ala
        195                 200                 205

Gly Thr Gly Gly Glu Lys Lys Arg Gly Gly Glu Glu Val Asn Lys Arg
    210                 215                 220

Glu Ala Val Met Leu Ala Glu Gly Pro Thr Glu Met Glu Glu Val Ala
225                 230                 235                 240

Val Gly Ala Pro Thr Pro Leu Ala Gly Ala Ala Gly Gly Pro His Ile
                245                 250                 255

Asp Arg Glu Tyr Ala Phe Arg Gln Glu Glu Glu Asp Leu His Val Ile
            260                 265                 270

Asp Ala Ser Ala Ser Val Glu Glu Val Ala Glu Glu Leu Trp Lys Val
        275                 280                 285

Val Ser Ala Arg Val Asp Ala Val Glu Arg Gly Glu Val Gly Lys Val
    290                 295                 300

Val Arg Arg Val Leu
305

<210> 4
<211> 210
<212> PRT
<213> Schizosaccharomyces pombe

<400> 4

Met Ser Lys Gln Asn Arg Gly Arg Leu Ile Val Ile Glu Gly Leu Asp
1               5                   10                  15

Arg Ser Gly Lys Ser Thr Gln Cys Gln Leu Leu Val Asp Lys Leu Ile
            20                  25                  30

Ser Gln His Glu Lys Ala Glu Leu Phe Lys Phe Pro Asp Arg Thr Thr
        35                  40                  45

Ala Ile Gly Lys Lys Ile Asp Asp Tyr Leu Lys Glu Ser Val Gln Leu
    50                  55                  60

Asn Asp Gln Val Ile His Leu Leu Phe Ser Ala Asn Arg Trp Glu Thr
65                  70                  75                  80

Ile Gln Tyr Ile Tyr Glu Gln Ile Asn Lys Gly Val Thr Cys Ile Leu
                85                  90                  95

Asp Arg Tyr Ala Phe Ser Gly Ile Ala Phe Ser Ala Ala Lys Gly Leu
            100                 105                 110

Asp Trp Glu Trp Cys Lys Ser Pro Asp Arg Gly Leu Pro Arg Pro Asp
        115                 120                 125

Leu Val Ile Phe Leu Asn Val Asp Pro Arg Ile Ala Ala Thr Arg Gly
    130                 135                 140

Gln Tyr Gly Glu Glu Arg Tyr Glu Lys Ile Glu Met Gln Glu Lys Val
145                 150                 155                 160

Leu Lys Asn Phe Gln Arg Leu Gln Lys Glu Phe Arg Glu Glu Gly Leu
                165                 170                 175

Glu Phe Ile Thr Leu Asp Ala Ser Ser Ser Leu Glu Asp Val His Ser
            180                 185                 190

Gln Ile Val Asp Leu Val Ser Asn Val Asn Ile His Glu Thr Leu Asp
        195                 200                 205

Val Leu
    210

<210>  5
<211>  216
<212>  PRT
<213>  Saccharomyces cerevisiae

<400>  5

Met Met Gly Arg Gly Lys Leu Ile Leu Ile Glu Gly Leu Asp Arg Thr
1               5                   10                  15

Gly Lys Thr Thr Gln Cys Asn Ile Leu Tyr Lys Lys Leu Gln Pro Asn
            20                  25                  30

Cys Lys Leu Leu Lys Phe Pro Glu Arg Ser Thr Arg Ile Gly Gly Leu
        35                  40                  45

Ile Asn Glu Tyr Leu Thr Asp Asp Ser Phe Gln Leu Ser Asp Gln Ala
    50                  55                  60

Ile His Leu Leu Phe Ser Ala Asn Arg Trp Glu Ile Val Asp Lys Ile
65                  70                  75                  80

Lys Lys Asp Leu Leu Glu Gly Lys Asn Ile Val Met Asp Arg Tyr Val
                85                  90                  95

Tyr Ser Gly Val Ala Tyr Ser Ala Ala Lys Gly Thr Asn Gly Met Asp
            100                 105                 110

Leu Asp Trp Cys Leu Gln Pro Asp Val Gly Leu Leu Lys Pro Asp Leu
        115                 120                 125

Thr Leu Phe Leu Ser Thr Gln Asp Val Asp Asn Asn Ala Glu Lys Ser
    130                 135                 140

Gly Phe Gly Asp Glu Arg Tyr Glu Thr Val Lys Phe Gln Glu Lys Val
145                 150                 155                 160

Lys Gln Thr Phe Met Lys Leu Leu Asp Lys Glu Ile Arg Lys Gly Asp
                165                 170                 175

Glu Ser Ile Thr Ile Val Asp Val Thr Asn Lys Gly Ile Gln Glu Val
            180                 185                 190

Glu Ala Leu Ile Trp Gln Ile Val Glu Pro Val Leu Ser Thr His Ile
        195                 200                 205

Asp His Asp Lys Phe Ser Phe Phe
    210                 215

<210>   6
<211>   212
<212>   PRT
<213>   Homo sapiens

<400>   6

Met Ala Ala Arg Arg Gly Ala Leu Ile Val Leu Glu Gly Val Asp Arg
1               5                   10                  15

Ala Gly Lys Ser Thr Gln Ser Arg Lys Leu Val Glu Ala Leu Cys Ala
            20                  25                  30

Ala Gly His Arg Ala Glu Leu Leu Arg Phe Pro Glu Arg Ser Thr Glu
        35                  40                  45

Ile Gly Lys Leu Leu Ser Ser Tyr Leu Gln Lys Lys Ser Asp Val Glu
    50                  55                  60

Asp His Ser Val His Leu Leu Phe Ser Ala Asn Arg Trp Glu Gln Val
65                  70                  75                  80

Pro Leu Ile Lys Glu Lys Leu Ser Gln Gly Val Thr Leu Val Val Asp
                85                  90                  95

Arg Tyr Ala Phe Ser Gly Val Ala Phe Thr Gly Ala Lys Glu Asn Phe
            100                 105                 110

Ser Leu Asp Trp Cys Lys Gln Pro Asp Val Gly Leu Pro Lys Pro Asp
        115                 120                 125

Leu Val Leu Phe Leu Gln Leu Gln Leu Ala Asp Ala Ala Lys Arg Gly
    130                 135                 140

Ala Phe Gly His Glu Arg Tyr Glu Asn Gly Ala Phe Gln Glu Arg Ala
145                 150                 155                 160

Leu Arg Cys Phe His Gln Leu Met Lys Asp Thr Thr Leu Asn Trp Lys
                165                 170                 175

Met Val Asp Ala Ser Lys Ser Ile Glu Ala Val His Glu Glu Leu Arg
            180                 185                 190

Val Leu Ser Glu Asp Ala Ile Arg Thr Ala Thr Glu Lys Pro Leu Gly
        195                 200                 205

Glu Leu Trp Lys
    210

<210>  7
<211>  227
<212>  PRT
<213>  Mus musculus

<400>  7

Met Ala Ser Arg Arg Gly Ala Leu Ile Val Leu Glu Gly Val Asp Arg
1               5                   10                  15

Ala Gly Lys Thr Thr Gln Gly Leu Lys Leu Val Thr Ala Leu Cys Ala
            20                  25                  30

Ser Gly His Arg Ala Glu Leu Leu Arg Phe Pro Glu Arg Ser Thr Glu
        35                  40                  45

Ile Gly Lys Leu Leu Asn Ser Tyr Leu Glu Lys Lys Thr Glu Leu Glu
    50                  55                  60

Asp His Ser Val His Leu Leu Phe Ser Ala Asn Arg Trp Glu Gln Val
65                  70                  75                  80

Pro Leu Ile Lys Ala Lys Leu Asn Gln Gly Val Thr Leu Val Leu Asp
                85                  90                  95

Arg Tyr Ala Phe Ser Gly Val Ala Phe Thr Gly Ala Lys Glu Asn Phe
            100                 105                 110

Ser Leu Asp Trp Cys Lys Gln Pro Asp Val Gly Leu Pro Lys Pro Asp
        115                 120                 125

Leu Ile Leu Phe Leu Gln Leu Gln Leu Leu Asp Ala Ala Ala Arg Gly
    130                 135                 140

Glu Phe Gly Leu Glu Arg Tyr Glu Thr Gly Thr Phe Gln Lys Gln Val

145                 150                 155                 160

Leu Leu Cys Phe Gln Gln Leu Met Glu Glu Lys Asn Leu Asn Trp Lys
                165                 170                 175

Val Val Asp Ala Ser Lys Arg Thr Pro Ser Glu Thr Leu His Arg Gly
            180                 185                 190

His Trp Gly Ser Tyr Gly Asn Lys Ser Ala Ser Ile Ala Asn Thr Ile
        195                 200                 205

Phe Trp Phe Cys Lys Arg Leu Val Glu Gly Ser His Leu Tyr Thr Ile
    210                 215                 220

Ser Arg Ser
225

<210>  8
<211>  212
<212>  PRT
<213>  Streptomyces pneumoniae

<400>  8

Met Ser Lys Gly Phe Leu Val Ser Leu Glu Gly Pro Glu Gly Ala Gly
1               5                   10                  15

Lys Thr Ser Val Leu Glu Ala Leu Leu Pro Ile Leu Glu Glu Lys Gly
            20                  25                  30

Val Glu Val Leu Thr Thr Arg Glu Pro Gly Gly Val Leu Ile Gly Glu
        35                  40                  45

Lys Ile Arg Glu Val Ile Leu Asp Pro Ser His Thr Gln Met Asp Ala
    50                  55                  60

Lys Thr Glu Leu Leu Leu Tyr Ile Ala Ser Arg Arg Gln His Leu Val
65                  70                  75                  80

Glu Lys Val Leu Pro Ala Leu Glu Ala Gly Lys Leu Val Ile Met Asp
                85                  90                  95

Arg Phe Ile Asp Ser Ser Val Ala Tyr Gln Gly Phe Gly Arg Gly Leu
            100                 105                 110

Asp Ile Glu Ala Ile Asp Trp Leu Asn Gln Phe Ala Thr Asp Gly Leu
        115                 120                 125

Lys Pro Asp Leu Thr Leu Tyr Phe Asp Ile Glu Val Glu Glu Gly Leu
    130                 135                 140

Ala Arg Ile Ala Ala Asn Ser Asp Arg Glu Val Asn Arg Leu Asp Leu
145                 150                 155                 160

Glu Gly Leu Asp Leu His Lys Lys Val Arg Gln Gly Tyr Leu Ser Leu
                165                 170                 175

Leu Asp Lys Glu Gly Asn Arg Ile Val Lys Ile Asp Ala Ser Leu Pro
            180                 185                 190

Leu Glu Gln Val Val Glu Thr Thr Asn Ala Val Leu Phe Asp Gly Met
        195                 200                 205

Gly Leu Ala Lys
    210

## Beschreibung

Die Erfindung bezieht sich auf ein Verfahren zum Identifizieren von Fungiziden, die Verwendung von Polypeptiden mit der enzymatischen Aktivität einer Thymidylat-Kinase zum Identifizieren von Fungiziden, und die Verwendung von Inhibitoren der Thymidylat-Kinase als Fungizide.

Unerwünschtes Pilzwachstum, das in der Landwirtschaft jedes Jahr zu beträchtlichen Schäden führt, kann durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von sogenannten Hochdurchsatzverfahren (HTS = high throughput screening). Dabei werden in einem automatisierten Verfahren eine große Anzahl von Einzelsubstanzen hinsichtlich ihrer Wirkung auf Zellen, individuelle Genprodukte oder Gene getestet. Wird für bestimmte Substanzen eine Wirkung nachgewiesen, so können diese in herkömmlichen Screeningverfahren untersucht und gegebenenfalls weiter entwickelt werden.

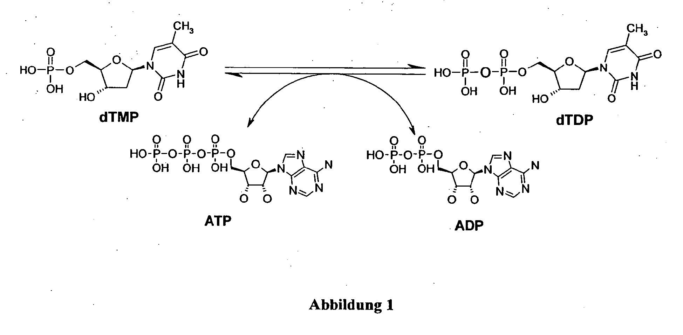

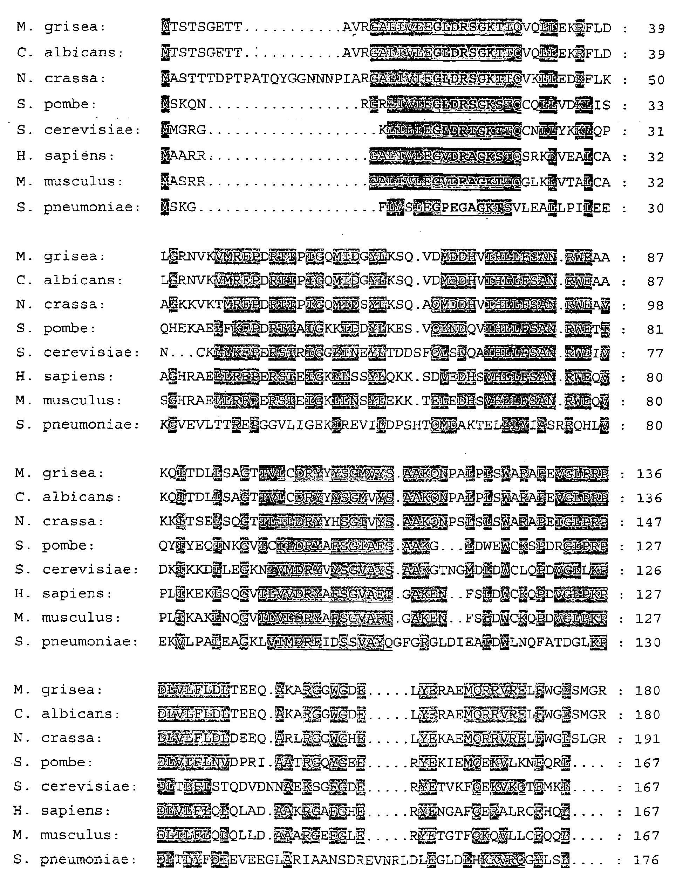

Die Thymidylat-Kinase (EC 2.7.4.9), auch bekannt als Thymidine-monophosphat-Kinase, Thymidylat-monophosphat-Kinase, Deoxythymidine-5'-monophosphate-Kinase, TMPK katalysiert die Reaktion von ATP mit Thymidin-5'-monophosphat (TMP) zu Thymidin-5'-diphosphate (TDP) (Phosphotransferase- Reaktion, siehe Abbildung 1).

Die von der Thymidylat-Kinase katalysierte Reaktion stellt einen essentiellen Schritt in der Bereitstellung von Vorstufen für die DNA-Synthese dar (Baranowska et al., 1990; Newlon and Fangman, 1975; Schild and Byers, 1978; Sclafani and Fangman, 1984; Zamb and Roth, 1977).

Gene für die Thymidylat-Kinase wurden aus den Pilzen *Saccharomyces cerevisiae* (Swissprot Accession No.: P00572) und *Schizosaccharomyces pombe* (Swissprot Accession No.: P36590) kloniert und konnte darüber hinaus auch in den Genomen weiterer Pilze (z.B. *Neurospora crassa* EAA29458) und pflanzenpathogener Pilze (z.B. *Magnaporthe grisea* MG09457.1) identifiziert werden. Daneben wurde die Thymidylat-Kinase auch aus zahlreichen anderen Organismen gewonnen, so z.B. aus *Homo sapiens* (Swissprot: Accession No.: P23919), *Arabidopsis thaliana* (Fragmant Swissprot: Accession No.: 081650) oder *Streptococcus pneumoniae* (Swissprot: Accession No.: Q8DQ58).

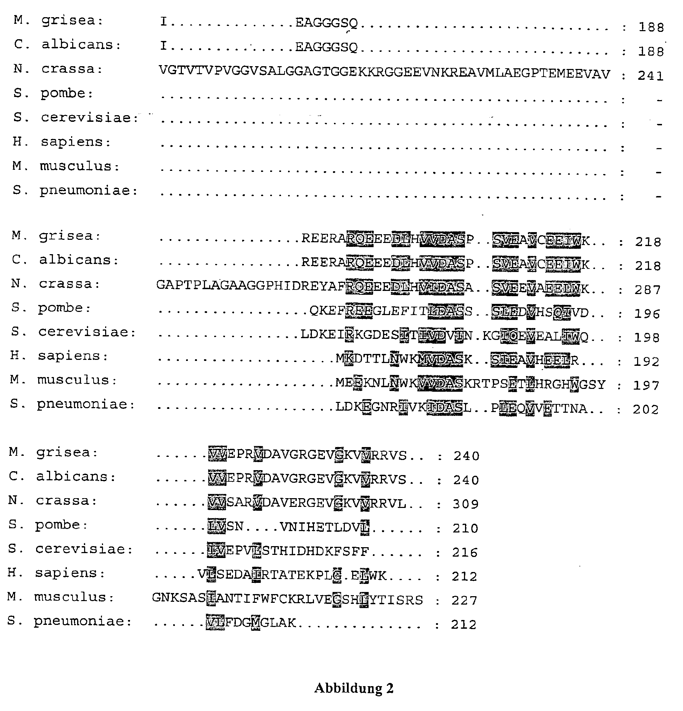

Die Sequenzähnlichkeiten sind innerhalb der eükaryontischen Klassen signifikant (40-50% Identität und 50-60% Ähnlichkeit), während hingegen die Sequenzidentität zu den bakteriellen Enzymen weniger signifikant ist (20-25% Identität und 40-50% Ähnlichkeit), siehe Abbildung 2.

Von den eukariotischen TMPK wurde das der Hefe (Lavie et al., 1998; Lavie et al., 1997) sowie das humane (Ostermann et al., 2000; Ostermann et al., 2003) Enzym in *E.coli* heterolog exprimiert und kristallisiert. Enzmycharakterisierungen erfolgten bislang aber ausschließlich mit gereinigtem Enzym aus z.B. *S. cerevisiae* (Jong and Campbell, 1984) oder *N. crassa* (Rossi and Wodward, 1975).

Aufgabe der vorliegenden Erfindung war es, neue Angriffspunkte von Fungiziden in Pilzen, insbesondere in phytopathogenen Pilzen, zu identifizieren und ein Verfahren zugänglich zu machen, in denen Inhibitoren eines solchen Angriffspunktes bzw. Polypeptids identifiziert und auf ihre fungiziden Eigenschaften hin geprüft werden können. Die Aufgabe wurde gelöst, indem aus einem phytopathogenen Pilz die für Thymidylat-Kinase kodierende Nukleinsäure isoliert, das davon kodierte Polypeptid gewonnen und ein Verfahren zur Verfügung gestellt wurde, mit dem Inhibitoren dieses Enzyms bestimmt werden können. Die mit diesem Verfahren identifizierten Inhibitoren können *in vivo* gegen Pilze eingesetzt werden.

### Beschreibung der Abbildungen

- **Abbildung 1:**: Schematische Darstellung der von der Thymidylat-Kinase katalysierten Reaktion. Die Thymidylat-Kinase katalysiert die Reaktion von Deoxythymidine 5'-phosphate (dTMP) mit Adenosine 5'-triphosphate (ATP) zu Deoxythymidine 5'-diphosphate (dTDP) und Adenosine 5'-diphosphate (ADP). Bei der Reaktion wird ein Molekül ATP und ein Molekül dTMP verbraucht.
- **Abbildung 2:**: Sequenzalignment von Thymidylat-Kinasen aus *Magnaporte grisea (M. grisea*), *Candida albicans* (*C. albicans*), *Neurospora crassa* (*N. crassa*), *Schizosaccharomyces pombe* (*S. pombe), Saccharomyces cerevisiae (S. cerevisiae*), *Homo sapiens* (*H. sapiens), Mus musculus* (*M. musculus)* und *Streptomyces pneumoniae* (*S. pneumoniae*). Graue Schattierung gibt den Bereich guter Konservierung an.

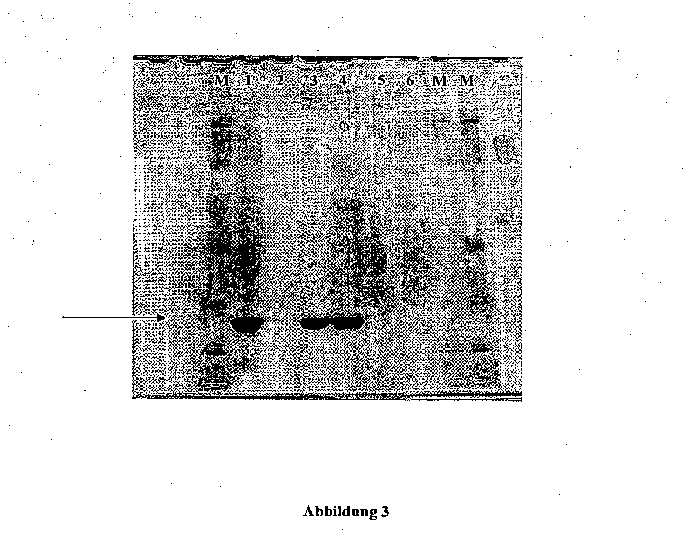

- **Abbildung 3:**: SDS-Gel zur Überprüfung der heterologen Expression der Thymidylat-Kinase in *E. coli* BL21(DE3)pLysS (Novagen). Das überexprimierte His₆-Fusionsprotein hat eine Grösse von 27,3 kDa. Jeweils Außen wurde ein Größenstandard aufgetragen (M). Spur 3 und 4: gereinigte Thymidylat-Kinase; Spur 1: Cytoplasmafraktion der überexprimierten Thymidylat-Kinase 24 Stunden nach der Induktion mit IPTG; Spur 5 und 6: Waschfraktionen nach dem Auftragen der Cytoplasmafraktion auf die Metall-Chelate-Sepharose Säule.

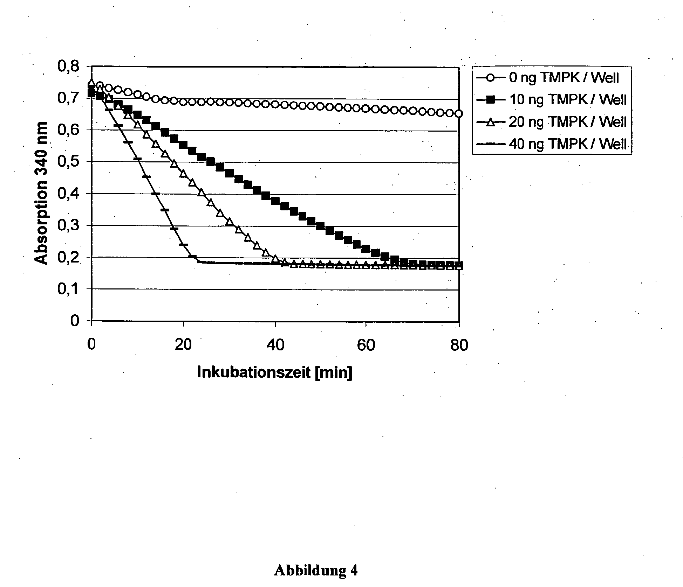

- **Abbildung 4:**: Graphische Darstellung der Kinetik der Umsetzung von Thymidylat und ATP durch unterschiedliche Konzentrationen an Thymidylat-Kinase im Assay. In einem Assayvolumen von 50 µl wurden 500 µM Thymidylat, 500 µM ATP, 300µM NADH, 400µM PEP (Phosphoenolpyruvat), 0,1U Pyruvate Kinase und 0,1U Lactate Dehydrogenase eingesetzt. Die verwendeten Proteinkonzentrationen der Thymidylat-Kinase sind der Abbildung zu entnehmen. Die Umsetzung wurde anhand der Absorptionsabnahme bei 340 nm durch die Reaktion des freigesetzten ADP mit PEP und NADH zu Lactat und NAD verfolgt.

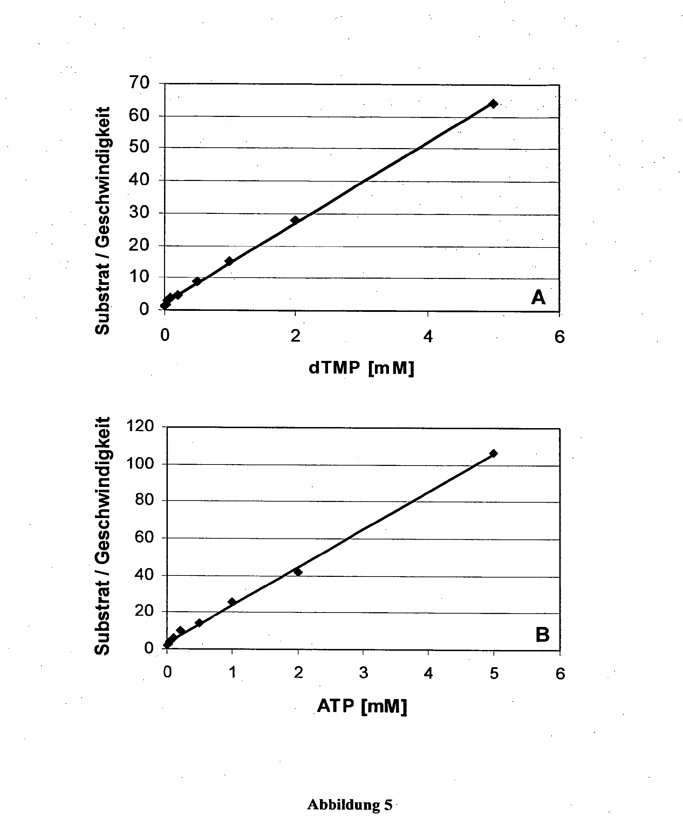

- **Abbildung 5:**: Lineweaver-Burk-Plot zur Bestimmung des K_{M}-Wertes von dTMP (A) und ATP (**B**). Die gemessenen Werte werden nach Lineweaver und Burk dargestellt, d.h. 1/Vₒ = 1/Vₘₐₓ + 1/S x (K_{M}/Vₘₐₓ), worin Vₒ die anfängliche Reaktionsgeschwindigkeit, Vₘₐₓ die maximal erreichbare Umsatzgeschwindigkeit und S die Substratkonzentarion ist. Vₘₐₓ und K_{M} lassen sich dann als Abszissen- und Ordinatenabschnitt 1/Vₘₐₓ bzw. 1/K_{M} ablesen. Der K_{M}-Wert für dTMP liegt bei 0,17 mM µM, der K_{M}-Wert für ATP liegt bei 0,16 mM µM.

### Definitionen

Unter dem Begriff "Homologie" bzw. "Identität" soll die Anzahl der übereinstimmenden Aminosäuren (Identität) mit anderen Proteinen, ausgedrückt in Prozent verstanden werden. Bevorzugt wird die Identität durch Vergleiche einer gegebenen Sequenz zu anderen Proteinen mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Die Identität kann standardmäßig mittels bekannten und der Öffentlichkeit zur Verfügung stehenden Computerprogrammen wie z.B. ClustalW (Thompson et al., *Nucleic Acids Research 22* (1994), 4673-4680) ermittelt werden. ClustalW wird z.B. öffentich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Génétique et de Biologie Moléculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK), heruntergeladen werden. Wenn das ClustalW Computerprogramm der Version 1.8 benutzt wird, um die Identität zwischen z.B. einem gegebenen Referenzprotein und anderen Proteinen zu bestimmen, sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP. Eine Möglichkeit zum Auffinden von ähnlichen Sequenzen ist die Durchführung von Sequenzdatenbankrecherchen. Hierbei wird eine oder werden mehrere Sequenzen als sogenannte Abfrage ("query") vorgegeben. Diese Abfragesequenz wird dann mittels statistischen Computerprogrammen mit Sequenzen, die in den ausgewählten Datenbanken enthalten sind, verglichen. Solche Datenbankabfragen ("blast searches") sind dem Fachmann bekannt und können bei verschiedenen Anbietern durchgeführt werden. Wird eine solche Datenbankabfrage z.B. beim NCBI (National Center for Biotechnology Information, http://www.ncbi.nlm.nih.gov/) durchgeführt, so sollen die Standardeinstellungen, die für die jeweilige Vergleichsanfrage vorgegeben sind, benutzt werden. Für Proteinsequenzvergleiche ("blastp") sind dieses folgende Einstellungen: Limit entrez = nicht aktiviert; Filter = low complexity aktiviert; Expect value = 10; word size = 3; Matrix = BLOSUM62; Gap costs: Existence = 11, Extension = 1. Als Ergebnis einer solchen Abfrage werden neben anderen Parametern auch der Anteil an Identität zwischen der Abfragesequenz und den in den Datenbanken aufgefundenen ähnlichen Sequenzen dargestellt. Unter einem erfindungsgemäßen Protein sollen daher im Zusammenhang mit der vorliegenden Erfindung solche Proteine verstanden werden, die bei der Verwendung mindestens einer der vorstehend beschriebenen Methoden zur Identitätsbestimmung eine Identität von mindestens 70 % aufweisen, bevorzugt von mindestens 75 %, besonders bevorzugt von mindestens 80 %, weiter bevorzugt von mindestens 85 %, und insbesondere von mindestens 90 %.

Der Ausdruck "vollständige Thymidylat-Kinase" wie er hierin verwendet wird, beschreibt eine Thymidylat-Kinase, die von einer vollständigen kodierenden Region einer Transkriptionseinheit kodiert wird, beginnend mit dem ATG-Startcodon und umfassend alle informationstragenden E-xonbereiche des im Herkunftsorganismus vorliegenden, für Thymidylat-Kinase kodierenden Gens, sowie die für eine korrekte Termination der Transkription nötigen Signale.

Der Ausdruck "biologische" oder "enzymatische" Aktivität einer Thymidylat-Kinase" wie er hierin verwendet wird, bezieht sich auf die Fähigkeit eines Polypeptids, die vorstehend beschriebene Reaktion, d.h. die Umsetzung von dTMP und ATP zu dTDP zu katalysieren.

Der Ausdruck "aktives Fragment" wie er hierin verwendet wird, beschreibt nicht mehr vollständige Nukleinsäuren kodierend für Thymidylat-Kinase, die aber noch für Polypeptide mit der enzymatische Aktivität einer Thymidylat-Kinase kodieren, und die eine für die Thymidylat-Kinase charakteristische Reaktion wie vorne beschrieben katalysieren können. Solche Fragmente sind kürzer als die oben beschriebenen vollständigen, für die Thymidylat-Kinase kodierenden Nukleinsäuren. Dabei können sowohl an den 3'- und/oder 5'-Enden der Sequenz Nukleinsäuren entfernt worden sein, es können aber auch Teile der Sequenz deletiert, d.h. entfernt worden sein, die die biologische Aktivität der Thymidylat-Kinase nicht entscheidend beeinträchtigen. Eine geringere oder gegebenenfalls auch eine erhöhte Aktivität, die aber noch die Charakterisierung bzw. Verwendung des resultierenden Thymidylat-Kinase Fragments gestattet, wird dabei als ausreichend im Sinne des hier verwendeten Ausdrucks verstanden. Der Ausdruck "aktives Fragment" kann sich ebenso auf die Aminosäuresequenz der Thymidylat-Kinase beziehen und gilt dann analog den obigen Ausführungen für solche Polypeptide, die im Vergleich zur oben definierten vollständigen Sequenz bestimmte Teile nicht mehr enthalten, wobei die biologische Aktivität des Enzyms jedoch nicht entscheidend beeinträchtigt ist. Die Fragmente können dabei verschiedene Länge besitzen.

Der Begriff "Thymidylat-Kinase-Hemmtest" oder "Hemmtest", wie er hierin verwendet wird, bezieht sich auf ein Verfahren bzw. einen Test, der es gestattet, die Inhibition der enzymatischen Aktivität eines Polypeptids mit der Aktivität einer Thymidylat-Kinase durch eine oder mehrere chemische Verbindungen (Kandidatenverbindung(en)) zu erkennen, wodurch die chemische Verbindung als Inhibitor der Thymidylat-Kinase identifiziert werden kann.

Der Ausdruck "Gen", wie er hierin verwendet wird, ist die Bezeichnung für einen Abschnitt aus dem Genom einer Zelle, der für die Synthese einer Polypeptid-Kette verantwortlich ist.

Der Ausdruck "Fungizid" bzw. "fungizid" wie er hierin verwendet wird, bezieht sich auf chemische Verbindungen, die zur Bekämpfung von Pilzen, insbesondere von pflanzenpathogenen Pilzen geeignet sind. Solche pflanzenpathogene Pilze werden nachfolgenden genannt, wobei die Aufzählung nicht abschließend ist:

Plasmodiophoromycetes, Oomycetes, Chytridiomycetes, Zygomycetes, Ascomycetes, Basidiomycetes und Deuteromycetes, z.B.

Pythium-Arten, wie beispielsweise *Pythium ultimum,* Phytophthora-Arten, wie beispielsweise *Phytophthora infestans*, Pseudoperonospora-Arten, wie beispielsweise *Pseudoperonospora humuli* oder *Pseudoperonospora cubensis,* Plasmopara-Arten, wie beispielsweise *Plasmopara viticola*, Bremia-Arten, wie beispielsweise *Bremia lactucae*, Peronospora-Arten, wie beispielsweise *Peronospora pisi* oder *P. brassicae*, Erysiphe-Arten, wie beispielsweise *Erysiphe graminis*, Sphaerotheca-Arten, wie beispielsweise *Sphaerotheca fuliginea*, Podosphaera-Arten, wie beispielsweise *Podosphaera leucotricha*, Venturia-Arten, wie beispielsweise *Venturia inaequalis*, Pyrenophora-Arten, wie beispielsweise *Pyrenophora teres* oder *P. graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus,* Puccinia-Arten, wie beispielsweise *Puccinia recondita*, Sclerotinia-Arten, wie beispielsweise *Sclerotinia sclerotiorum,* Tilletia-Arten, wie beispielsweise *Tilletia caries*; Ustilago-Arten, wie beispielsweise *Ustilago nuda* oder *Ustilago avenae*, Pellicularia-Arten, wie beispielsweise *Pellicularia sasakii*, Pyricularia-Arten, wie beispielsweise *Pyricularia oryzae*, Fusarium-Arten, wie beispielsweise *Fusarium culmorum,* Botrytis-Arten, Septoria-Arten, wie beispielsweise *Septoria nodorum,* Leptosphaeria-Arten, wie beispielsweise *Leptosphaeria nodorum,* Cercospora-Arten, wie beispielsweise *Cercospora canescens*, Alternaria-Arten, wie beispielsweise *Alternaria brassicae* oder Pseudocercosporella-Arten, wie beispielsweise *Pseudocercosporella herpotrichoides.*

Von besonderem Interesse sind z.B. auch *Magnaporthe grisea, Cochliobulus heterostrophus, Nectria hematococcus* and Phytophtora species.

Fungizide Wirkstoffe, die mit Hilfe der erfindungsgemäßen Thymidylat-Kinase gefunden werden, können aber auch mit Thymidylat-Kinasen aus humanpathogenen Pilzspezies interagieren, wobei die Interaktion mit den unterschiedlichen in diesen Pilzen vorkommenden Thymidylat-Kinase nicht immer gleich stark sein muss.

Dabei sind die folgenden humanpathogenen Pilze von besonderem Interesse, die unter anderem die nachfolgend genannten Krankheitsbilder hervorrufen können:
Dermatophyten, wie z.B. Trichophyton spec., Microsporum spec., *Epidermophyton floccosum* oder *Keratomyces ajelloi,* die z.B. Fußmykosen (Tinea pedis) hervorrufen,
Hefen, wie z.B. *Candida albicans,* Soor-Ösophagitis und Dermatitis hervorruft, *Candida glabrata, Candida krusei* oder *Cryptococcus neoformans,* die z.B. pulmonale Cryptococcose und auch Torulose hervorrufen können,
Schimmelpilze, wie z.B. *Aspergillus fumigatus, A. flavus, A. niger,* die z.B. bronchopulmonale Aspergillose oder Pilzsepsis hervorrufen, Mucor spec., Absidia spec., oder Rhizopus spec., die z.B. Zygomykosen (intravasale Mykosen) hervorrufen, *Rhinosporidium seeberi,* der z.B. chronische granulomatöse Pharyngitis und Tracheitis hervorruft, *Madurella myzetomatis*, der z.B. subkutane Myzetome hervorruft, *Histoplasma capsulatum,* der z.B. retikuloendotheliale Zytomykose und M. Darling hervorruft, *Coccidioides immitis,* der z. B. pulmonale Coccidioidomykose und Sepsis hervorruft, *Paracoccidioides brasiliensis*, der z.B. brasilianische Blastomykose hervorruft, *Blastomyces dermatitidis*, der z.B. Gilchrist-Krankheit und nordamerikanische Blastomykose hervorruft, *Loboa loboi,* der z.B. Keloid-Blastomykose und Lobo's Krankheit hervorruft, und *Sporothrix schenckii*, der z.B. Sporotrichose (granulomatöse Hautmykose) hervorruft.

Im Folgenden sollen die Begriffe "fungizid" bzw. "Fungizid" gleichermaßen für die Begriffe "antimykotisch" bzw. "Antimykotikum" als auch für die Begriffe "fungizid" bzw. "Fungizid" im herkömmlichen Sinne, d.h. bezogen auf pflanzenpathogene Pilze, verwendet werden. Fungizide Wirkstoffe, die mit Hilfe einer aus einem bestimmten Pilz, hier z.B. aus *S. cerevisiae*, gewonnenen Thymidylat-Kinase gefunden werden, können also auch mit einer Thymidylat-Kinase aus zahlreichen anderen Pilzspezies, gerade auch mit pflanzenpathogenen Pilzen interagieren, wobei die Interaktion mit den unterschiedlichen in diesen Pilzen vorkommenden Thymidylat-Kinasen nicht immer gleich stark sein muss. Dies erklärt unter anderem die beobachtete Selektivität der an diesem Enzym wirksamen Substanzen.

Der Ausdruck "Agonist", wie er hierin verwendet wird, bezieht sich auf ein Molekül, das die Aktivität der Thymidylat-Kinase beschleunigt oder verstärkt.

Der Ausdruck "Antagonist", wie er hierin verwendet wird, bezieht sich auf ein Molekül, das die Aktivität der Thymidylat-Kinase verlangsamt oder verhindert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden bzw. die deren Aktivität beeinflussen. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Liganden darstellen oder strukturelle oder funktionelle Mimetika davon. Bevorzugt handelt es sich beim Ausdruck "Modulator", wie er hierin verwendet wird, jedoch um solche Moleküle, die nicht die natürlichen Substrate bzw. Liganden darstellen.

### Beschreibung der Erfindung

Trotz umfangreicher Forschungen an der Thymidylat-Kinase war bislang unbekannt, dass die Thymidylat-Kinase in phytopathogenen Pilzen ein Zielprotein (ein so genanntes "Target") fungizid wirksamer Substanzen sein kann.

Für bereits bekannte Inhibitoren der viralen Thymidylat-Kinase wurde keine fungizide Wirkung beschrieben (siehe z.B. Bone et al., 1986; Davies et al., 1988; Haouz et al., 2003; Manallack et al., 2002).

Damit wird in der vorliegenden Erfindung zum ersten Mal gezeigt, dass die Thymidylat-Kinase ein auch für Pilze wichtiges Enzym darstellt und deshalb in besonderem Maße dazu geeignet ist, als Zielprotein für die Suche nach neuen und verbesserten fungizid wirksamen Wirkstoffen verwendet zu werden.

Im Rahmen der vorliegenden Erfindung konnte mit Hilfe von Knock-out Experimenten gezeigt werden (Beispiel 1), dass die Thymidylat-Kinase ein Angriffspunkt oder "Target" für fungizide Wirkstoffe sein kann, die Inhibition der Thymidylat-Kinase also zur Schädigung oder zum Absterben des Pilzes führen könnte. Es konnte weiterhin gezeigt werden , dass das Enzym Thymidylat-Kinase zum Identifizieren von Modulatoren bzw. Inhibitoren seiner enzymatischen Aktivität in Testverfahren verwendet werden kann, was bei verschiedenen theoretisch interessanten Targets, die z.B. bereits als essentiell für einen Organismus bekannt sind, nicht selbstverständlich ist. Schließlich konnte auch gezeigt werden, dass Inhibitoren der Thymidylat-Kinase, die in solchen Verfahren identifiziert werden konnten, als Fungizide eingesetzt werden können.

Im Rahmen der vorliegenden Erfindung wurde ein Verfahren entwickelt, das geeignet ist, die Aktivität der Thymidylat-Kinase sowie die Hemmung dieser Aktivität in einem so genannten Hemmtest zu bestimmen und auf diese Weise Inhibitoren des Enzyms z.B. in HTS- und UHTS-Verfahren zu identifizieren. Die mit Hilfe dieses erfindungsgemäßen Verfahrens identifizierten Verbindungen können in *in vivo* Tests an Pilzen geprüft werden.

Im Rahmen der vorliegenden Erfindung wurde so gefunden, dass die Thymidylat-Kinase auch *in vivo* durch Wirkstoffe inhibiert werden und ein mit diesen Wirkstoffen behandelter pilzlicher Organismus durch die Behandlung mit diesen Wirkstoffen geschädigt und abgetötet werden kann. Die Inhibitoren einer pilzlichen Thymidylat-Kinase können also als Fungizide insbesondere im Pflanzenschutz oder auch als Antimykotika in Pharmaindikationen verwendet werden. In der vorliegenden Erfindung wird beispielsweise gezeigt, dass die Hemmung der Thymidylat-Kinase mit einer in einem erfindungsgemäßen Verfahren identifizierten Substanzen zum Absterben oder zu einer Wachstumshemmung der behandelten Pilze in synthetischen Medien bzw. auf der Pflanze führt.

Die Thymidylat-Kinase kann z.B. in einfacher Weise aus Pilzen wie *S. cerevisiae* gewonnen werden. Zur Herstellung der Thymidylat-Kinase aus Hefe kann das Gen z.B. rekombinant in *Escherichia coli* exprimiert und aus *E. coli* Zellen eine Enzympräparation hergestellt werden (Beispiel 2).

So wurde für die Expression des von *cdc8* kodierten Polypeptids CDC8 aus Hefe (Jong and Campbell, 1984; SWISS-PROT Accession Nummer: P00572) der zugehörige ORF aus genomischer DNA nach dem Fachmann bekannten Methoden über genspezifische Primer amplifiziert. Die entsprechende DNA wurde in den Vektor pENTR/D-TOPO (Invitrogen Corporation, Carlsbad, California, USA) kloniert und mittels spezifischer Rekombination (Gateway-Technologie Invitrogen) in den Vektor pDest17 (Invitrogen) transferiert. Das resultierende Plasmid pDest17-cdc8 enthält die vollständige kodierende Sequenz von cdc8 in einer N-terminalen Fusion mit einem His6-Tag aus dem Vektor. Das CDC8 Fusionsprotein besitzt eine berechnete Masse von 27 kDa (vgl. Beispiel 2 und Abbildung 3).

Das Plasmid pDEST17-cdc8 wurde dann zur rekombinanten Expression von CDC8 in *E. coli* BL21(DE3)pLysS (Novagen Inc.) Zellen verwendet (vgl. Beispiel 2).

Wie bereits vorstehend ausgeführt, ist die vorliegende Erfindung nicht nur auf die Verwendung von Thymidylat-Kinase aus Hefe beschränkt. In analoger und dem Fachmann bekannter Weise können auch aus anderen Pilzen, vorzugsweise aus pflanzenpathogenen Pilzen, Polypeptide mit der Aktivität einer Thymidylat-Kinase gewonnen werden, die dann in einem erfindungsgemäßen Verfahren eingesetzt werden können. Bevorzugt wird z.B. die Thymidylat-Kinase aus *S. cerevisiae* verwendet.

Thymidylat-Kinasen teilen sich homologe Bereiche anhand derer sie identifiziert werden können. Typisch für Thymidylat-Kinase ist ein Motiv, das sich bei vielen ATP-bindenden Enzymen findet. Es handelt sich bei diesem konservierten Motiv um eine Glycin-reiche Region, die typischerweise eine flexible Schleife (einen so genannten "Loop") zwischen einem Beta-Faltblatt und einer Alpha-Helix bildet. Diese Schleife interagiert mit einer der Phosphatgruppen des Nukleotids. Dieses Sequenzmotiv wird im Allgemeinen als "P-Loop" bezeichnet und entspricht der folgenden Sequenz, die in Abbildung 2 unterstrichen dargestellt ist :

[AG]-x(4)-G-K-[ST]

Vorzugsweise werden Thymidylat-Kinasen mit dem Sequenzmotiv G-L-D-R-x-G-K-T verwendet.

Es ist auch möglich, ein weiteres für Thymidylat-Kinasen typisches Motiv anzugeben, dass eine Identifizierung dieses Enzyms neben Enzymtests ermöglicht (Hofmann K., Bucher P., Falquet L., Bairoch A. (1999) "The PROSITE database, its status in 1999". *Nucleic Acids Res.* 27, 215). Es kann wie folgt dargestellt werden:

[LIV]-[LIVMGSTC]-[DET]-[RH]-[FYHCS]-[]x(2)-S-[GSTNP]-w-[AVC]-[FY]-[STANQ].

Vorzugsweise werden Thymidylat-Kinasen mit dem Sequenzmotiv [LIV]-[LIVMGSTC]-D-R-Y-x(2)-S-G-x-[AV]-[FY]-S verwendet. Das Motiv ist in Abbildung 2 unterstrichen.

PROSITE ermöglicht Polypeptiden eine Funktion zuzuordnen und somit Thioredoxin-Reduktasen als solche zu erkennen. Bei der Darstellung des Prosite Motivs wird der "Ein-Buchstaben-Code" verwendet. Das Symbol "x" steht für eine Position, an der jede Aminosäure akzeptiert wird. Eine variable Position, an der verschiedene bestimmte Aminosäuren akzeptiert werden, wird in eckigen Klammern "[...]" dargestellt, wobei die an dieser Position möglichen Aminosäuren aufgezählt werden. Aminosäuren, die an einer bestimmten Position nicht akzeptiert werden, stehen dagegen in geschweiften Klammern "{...}". Ein Gedankenstrich "-" trennt die einzelnen Elemente bzw. Positionen des Motivs. Wiederholt sich eine bestimmte Position, z.B. "x", mehrfach hintereinander, kann dies durch Angabe der Zahl der Wiederholungen in einer nachfolgenden Klammer dargestellt werden, z.B. "x (3)", was für "x-x-x" steht.

Ein Prosite Motiv stellt also letztlich die Komponenten einer Konsensussequenz dar, sowie Abstände zwischen den beteiligten Aminosäuren und ist damit typisch für eine bestimmte Enzymklasse. Anhand dieses Motivs können auf Basis bekannter für Thymidylat-Kinasen kodierender Nukleinsäuren weitere Polypeptide aus (pflanzenpathogenen) Pilzen indentifiziert bzw. zugeordnet werden, die zur selben Klasse wie das erfindungsgemäße Polypeptid gehören und deshalb auch in erfindungsgemäßer Weise verwendet werden können.

Im Falle der Thymidylat-Kinase aus *S. cerevisiae* liegt dieses Motiv ebenso vor wie bei *S. pombe, M. grisea, C. albicans* oder *N. crassa* (vgl. Abbildung 2).

Das oben genannte Prosite Motiv bzw. die spezifische Konsensussequenz sind typisch für die erfindungsgemäßen Polypeptide, die anhand dieser Konsensussequenzen strukturell definiert werden können und damit auch eindeutig identifizierbar sind.

Gegenstand der vorliegenden Erfindung sind deshalb auch Polypeptide aus pflanzenpathogenen Pilzen mit der enzymatischen Aktivität einer Thymidylat-Kinase, die das vorstehend genannte Prosite Motiv [LIV]-[LIVMGSTC]-[DET]-[RH]-[FYHCS]-x(2)-S-[GSTNP]-x-[AVC]-[FY]-[STANQ] und das Motiv des P-Loops, [AG]-x(4)-G-K-[ST], umfassen.

Es ist dem Fachmann möglich, z.B. mittels PCR weitere für Thymidylat-Kinasen kodierende Nukleinsäuren aus anderen (pflanzenpathogenen) Pilzen zu erhalten und zu identifizieren. Solche Nukleinsäuren und deren Verwendung in Verfahren zum Identifizieren von fungiziden Wirkstoffen werden als von der vorliegenden Erfindung umfasst betrachtet.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise am Peptid-Rückgrat, an der Aminosäure-Seitenkette, am Amino- und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezemierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen. Die erfindungsgemäßen Proteine können ebenfalls so vorliegen, wie sie natürlicherweise in ihrem Herkunftsorganismus vorliegen, aus dem sie zum Beispiel direkt gewonnen werden können. In den erfindungsgemäßen Verfahren können ebenso aktive Fragmente einer Thymidylat-Kinase eingesetzt werden, solange sie die Bestimmung der enzymatischen Aktivität des Polypeptids bzw. deren Inhibition durch eine Kandidatenverbindung ermöglichen.

Die in den erfindungsgemäßen Verfahren eingesetzten Polypeptide können im Vergleich zu den entsprechenden Regionen von natürlich vorkommenden Thymidylat-Kinasen Deletionen oder A-minosäuresubstitutionen aufweisen, solange sie zumindest noch die biologische Aktivität einer vollständigen Thymidylat-Kinase zeigen. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Ein mögliches Reinigungsverfahren der Thymidylat-Kinase basiert auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie oder Affinitätschromatographie (vgl. Beispiel 2).

Ein schnelles Verfahren zum Isolieren von Thymidylat-Kinasen, die von Wirtszellen synthetisiert werden, beginnt mit der Expression eines Fusionsproteins, wobei der Fusionspartner auf einfache Weise affinitätsgereinigt werden kann. Der Fusionspartner kann beispielsweise ein His6-Tag sein (vgl. Beispiel 2). Das Fusionsprotein kann dann an Ni-NTA-Agarose gereinigt werden. Der Fusionspartner kann durch partielle proteolytische Spaltung beispielsweise an Linkem zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren wiederum auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie und Affinitätschromatographie.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Das Verfahren zum Herstellen von Polypeptiden mit der Aktivität einer Thymidylat-Kinase, wie z.B. des Polypeptids CDC8, ist damit gekennzeichnet durch
(a) das Kultivieren einer Wirtszelle enthaltend zumindest eine exprimierbare Nukleinsäuresequenz kodierend für ein Polypeptid aus Pilzen mit der biologischen Aktivität einer Thymidylat-Kinase unter Bedingungen, die die Expression dieser Nukleinsäure gewährleisten, oder
(b) das Exprimieren einer exprimierbaren Nukleinsäuresequenz kodierend für ein Polypeptid aus Pilzen mit der biologischen Aktivität einer Thymidylat-Kinase in einem *in vitro-*-System, und
(c) die Gewinnung des Polypeptids aus der Zelle, dem Kulturmedium oder dem *in vitro-*-System.

Die so erhaltenen Zellen enthaltend das erfindungsgemäße Polypeptid oder das so erhaltene gereinigte Polypeptid sind geeignet, in Verfahren zum Identifizieren von Modulatoren bzw. Inhibitoren der Thymidylat-Kinase verwendet zu werden.

Gegenstand der vorliegenden Erfindung ist auch die Verwendung von Polypeptiden aus Pilzen, welche die enzymatische Aktivität einer Thymidylat-Kinase ausüben, in Verfahren zum Identifizieren von Inhibitoren von Polypeptiden mit der Aktivität einer Thymidylat-Kinase, wobei die Inhibitoren der Thymidylat-Kinase als Fungizide verwendet werden können. Besonders bevorzugt wird die Thymidylat-Kinase aus *S. cerevisiae* verwendet.

Fungizide Wirkstoffe, die mit Hilfe einer Thymidylat-Kinase aus einer bestimmten Pilzspezies gefunden werden, können auch mit Thymidylat-Kinasen aus anderen Pilzspezies interagieren, wobei die Interaktion mit den unterschiedlichen in diesen Pilzen vorkommenden Thymidylat-Kinase nicht immer gleich stark sein muss. Dies erklärt unter anderem die Selektivität wirksamer Substanzen. Die Verwendung von Wirkstoffen, die mit einer Thymidylat-Kinase einer bestimmten PilzSpezies gefunden wurden, als Fungizide auch bei anderen Pilze-Spezies kann darauf zurückgeführt werden, dass sich Thymidylat-Kinasen aus verschiedenen Pilzspezies sehr nahe stehen und in größeren Bereichen eine ausgeprägte Homologie zeigen. So wird an Abbildung 2 deutlich, dass eine solche Homologie über beträchtliche Sequenzabschnitte hinweg zwischen *S. cerevisiae, N. crassa und S. pombe* besteht und damit die Wirkung der mit Hilfe der Thymidylat-Kinase aus Hefe gefundenen Substanzen nicht auf *S. cerevisiae* beschränkt bleibt. In Verfahren zum Identifizieren von Fungiziden werden deshalb bevorzugt Polypeptide mit der enzymatischen Aktivität einer Thymidylat-Kinase verwendet, die eine Konsensussequenz gemäß Abbildung 2 aufweisen.

Gegenstand der vorliegenden Erfindung ist deshalb auch ein Verfahren zum Identifizieren von Fungiziden durch Testen von potentiellen Inhibitoren bzw. Modulatoren der enzymatischen Aktivität der Thymidylat-Kinase (Testverbindung) in einem Thymidylat-Kinase-Hemmtest (Beispiel 3).

Verfahren, die geeignet sind, Modulatoren, insbesondere Inhibitoren bzw. Antagonisten der erfindungsgemäßen Polypeptide zu identifizieren, beruhen in aller Regel auf der Bestimmung der Aktivität bzw. der biologischen Funktionalität des Polypeptids. Dazu kommen prinzipiell sowohl auf ganzen Zellen beruhende Verfahren *(in vivo* Verfahren) in Frage, wie auch Verfahren, die auf der Verwendung des aus den Zellen isolierten Polypeptids beruhen, das in gereinigter oder teilweise gereinigter Form oder auch als Rohextrakt vorliegen kann. Diese zellfreien *in vitro* Verfahren können ebenso wie *in vivo* Verfahren im Labormaßstab, in bevorzugter Weise aber auch in HTS oder UHTS Verfahren genutzt werden. Im Anschluss an die *in vivo* oder *in vitro* Identifizierung von Modulatoren des Polypeptids können Tests an Pilzkulturen durchgeführt werden, um die fungizide Wirksamkeit der gefundenen Verbindungen zu verifizieren.

Viele Testsysteme, die die Prüfung von Verbindungen und natürlichen Extrakten zum Ziel haben, sind bevorzugt auf hohe Durchsatzzahlen ausgerichtet, um die Zahl der untersuchten Substanzen in einem gegebenen Zeitraum zu maximieren. Testsysteme, die auf zellfreiem Arbeiten beruhen, brauchen gereinigtes oder semi-gereinigtes Protein. Sie sind geeignet für eine "erste" Prüfung, die in erster Linie darauf abzielt, einen möglichen Einfluss einer Substanz auf das Zielprotein zu detektieren. Ist eine solche erste Prüfung erfolgt und eine oder mehrere Verbindungen, Extrakte etc. gefunden, kann die Wirkung solcher Verbindungen im Labor noch gezielter untersucht werden. So kann in einem ersten Schritt die Inhibierung oder Aktivierung des erfindungsgemäßen Polypeptids *in vitro* noch einmal geprüft werden, um im Anschluss daran die Wirksamkeit der Verbindung am Zielorganismus, hier einem oder mehreren pflanzenpathogenen Pilzen, zu testen. Die Verbindung kann dann gegebenenfalls als Ausgangspunkt für die weitere Suche und Entwicklung von fungiziden Verbindungen verwendet werden, die auf der ursprünglichen Struktur basieren, jedoch z.B. hinsichtlich Wirksamkeit, Toxizität oder Selektivität optimiert sind.

Um Modulatoren aufzufinden, kann z.B. ein synthetischer Reaktionsmix (z.B. Produkte der *in vitro* Transkription) oder ein zellulärer Bestandteil, wie eine Membran, ein Kompartiment oder irgendeine andere Präparation, die die erfindungsgemäßen Polypeptide enthält, zusammen mit einem gegebenenfalls markierten Substrat oder Liganden der Polypeptide in Gegenwart und Abwesenheit einer Testverbindung (oder auch eines Gemisches von Testverbindungen), die ein Antagonist sein kann, inkubiert werden. Die Fähigkeit der Testverbindung, die Aktivität der Thymidylat-Kinase zu hemmen, wird z.B. erkennbar an einer verringerten Umsetzung des gegebenenfalls markierten Substrates.

Die Detektion der biologischen Aktivität der erfindungsgemäßen Polypeptide kann durch ein so genanntes Reportersystem verbessert werden. Reportersysteme in dieser Hinsicht umfassen, sind aber nicht beschränkt auf colorimetrisch oder fluorimetrische nachweisbare Substrate, die in ein Produkt umgewandelt werden oder ein Reportergen, das auf Veränderungen der Aktivität oder der Expression der erfindungsgemäßen Polypeptide anspricht oder andere bekannte Bindungstests.

Ein weiteres Beispiel für ein Verfahren, mit welchem Modulatoren der erfindungsgemäßen Polypeptide aufgefunden werden können, ist ein Verdrängungstest, bei dem man unter dafür geeigneten Bedingungen die erfindungsgemäßen Polypeptide und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt. Die erfindungsgemäßen Polypeptide selbst können markiert werden, z.B. fluorimetrisch oder colorimetrisch, so dass man die Anzahl der Polypeptide, die an einen Liganden gebunden sind oder die eine Umsetzung mitgemacht haben, exakt bestimmen kann. Ebenso kann jedoch die Bindung mittels des gegebenenfalls markierten Substrats, Liganden bzw. Substratanalogen verfolgt werden. Auf diese Weise lässt sich die Effektivität von Antagonisten ermessen.

Effekte wie Zelltoxizität werden in diesen *in vitro* Systemen in der Regel ignoriert. Die Testsysteme überprüfen dabei sowohl inhibierende bzw. suppressive Effekte der Substanzen, als auch stimulatorische Effekte. Die Effektivität einer Substanz kann durch konzentrationsabhängige Testreihen überprüft werden. Kontrollansätze ohne Testsubstanzen bzw. ohne Enzym können zur Bewertung der Effekte herangezogen werden.

Eine andere Möglichkeit zur Identifizierung von Substanzen, die die Aktivtät der erfindungsgemäßen Polypeptide modulieren, ist so auch der sogenannten "Scintillation Proximity Assay" (SPA), siehe EP 015 473. Dieses Testsystem nutzt die Interaktion eines Polypeptids (z.B. Thymidylat-Kinase aus Hefe) mit einem radiomarkierten Liganden bzw. Substrat. Das Polypeptid ist dabei an kleine Kügelchen ("Microspheres") oder Perlen ("Beads") gebunden, die mit szintillierenden Molekülen versehen sind. Im Verlauf des Abfalls der Radioaktivität wird die szintillierende Substanz im Kügelchen durch die subatomaren Partikel des radioaktiven Markers angeregt und ein detektierbares Photon emittiert. Die Testbedingungen werden so optimiert, dass nur jene vom Liganden ausgehenden Partikel zu einem Signal führen, die von einem an das erfindungsgemäße Polypeptid gebundenen Liganden ausgehen.

Vorzugsweise handelt es sich bei den zu identifizierenden Modulatoren um kleine organisch-chemische Verbindungen.

Ein Verfahren zum Identifizieren einer Verbindung, die die Aktivität einer Thymidylat-Kinase aus Pilzen moduliert und die als Fungizid im Pflanzenschutz verwendet werden kann, besteht daher darin, dass man
a) ein Polypeptid mit der enzymatischen Aktivität einer Thymidylat-Kinase, vorzugsweise aus Pilzen, insbesondere bevorzugt aus pflanzenpathogenen Pilzen, oder eine Wirtszelle enthaltend eine solches Poylpeptid mit einer chemischen Verbindung oder mit einem Gemisch von chemischen Verbindungen unter Bedingungen in Kontakt bringt, die die Interaktion einer chemischen Verbindung mit dem Polypeptid erlauben,
b) die Aktivität des Polypeptids bei Abwesenheit einer chemischen Verbindung mit der Aktivität des erfindungsgemäßen Polypeptids bei Anwesenheit einer chemischen Verbindung oder eines Gemisches von chemischen Verbindungen vergleicht, und
c) die chemische Verbindung bestimmt, die die Aktivität des erfindungsgemäßen Polypeptids spezifisch moduliert und
d) gegebenenfalls die fungizide Wirkung der bestimmten Verbindung *in vivo* prüft.

Besonders bevorzugt wird dabei diejenige Verbindung bestimmt, die die Aktivität des erfmdungsgemäßen Polypeptids spezifisch inhibiert. Der Begriff "Aktivität", wie er hier verwendet wird, bezieht sich auf die biologische Aktivität des erfindungsgemäßen Polypeptids.

Die auf diese Weise identifizierten Verbindungen können als Fungizide zur Bekämpfung von Pilzen, bevorzugt von pflanzenpathogenen Pilzen verwendet werden. Gegenstand der vorliegenden Erfindung ist daher auch ein Verfahren zum Identifizieren von Fungiziden, in dem man
a) die vorstehend beschriebenen Schritte (a) bis (d) durchführt,
b) die identifizierte fungizide Verbindung in geeigneter Weise formuliert, und
c) die identifizierte Verbindung auf den zu bekämpfenden Pilz und/oder seinen Lebensraum einwirken lässt.

Ein bevorzugtes Verfahren (siehe Beispiel 3) nutzt die Tatsache aus, dass bei der Reaktion der Thymidylat-Kinase ein Molekül Adenosindiphosphat (ADP) freigesetzt wird. Die Aktivität bzw. die Ab- oder Zunahme der Aktivität des erfindungsgemäßen Polypeptids kann deswegen durch den Nachweis des entstehenden ADP bestimmt werden. Dabei wird die geringere bzw. inhibierte Aktivität der betreffenden Thymidylat-Kinase, die sich durch die verminderte Entstehung von ADP ausdrückt, anhand des Nachweises des entstehenden ADP durch Kopplung an die nachgeschaltete Reaktion der Pyruvatkinase und Laktatdehydrogenase verfolgt. Die Pyruvatkinase setzt dabei unter Verbrauch des entstehenden ADP gemäß der Gleichung *ADP + Phosphoenolpyruvat ⇔ ATP + Pyruvat* Phosphoenolpyruvat zu Pyruvat um, welches dann von der Laktat Dehydrogenase zur Oxidation von NADH zu NAD verwendet wird. Die durch die gekoppelte Reaktion steigende NAD-Konzentration bzw. abnehmende NADH-Konzentration kann photospektrometrisch durch Absorptions- oder Fluoreszenzmessung (bei 340 nm bzw. einer Anregungswellenlänge von 360 nm und einer Emissionswellenlänge von 465 nm) bestimmt werden.

Die Messung kann auch in für HTS- oder UHTS-Assays gängigeren Formaten erfolgen, z.B. in Mikrotiterplatten, in denen z.B. ein Gesamtvolumen von 5 bis 50 µl pro Ansatz bzw. pro Well vorgelegt wird und die einzelnen Komponenten in der gewünschten Endkonzentrationen vorliegen (vgl. Beispiel 3). Dabei wird die zu testende, potentiell die Aktivität des Enzyms inhibierende oder aktivierende Verbindung (Kandidatenmolekül) z.B. in einer geeigneten Konzentration in Testpuffer enthaltend dTMP, Adenosintriphosphat, Phosphoenolpyruvat und die gekoppelten Hilfsenzyme vorgelegt. Dann wird das erfindungsgemäße Polypeptid in Testpuffer zugegeben und die Reaktion damit gestartet. Der Ansatz wird dann z.B. bis zu 30 Minuten bei einer geeigneten Temperatur inkubiert und die Absorptionsabnahme bei 340 nm gemessen.

Eine weitere Messung erfolgt in einem entsprechenden Ansatz, jedoch ohne Zugabe eines Kandidatenmoleküls (Testverbindung) und ohne Zugabe von Thymidylat-Kinase (Negativkontrolle). Eine weitere Messung erfolgt wiederum bei Abwesenheit eines Kandidatenmoleküls, jedoch bei Anwesenheit von Thymidylat-Kinase (Positivkontrolle). Negativ- und Positivkontrolle ergeben damit die Vergleichswerte zu den Ansätzen bei Anwesenheit eines Kandidatenmoleküls.

Um optimale Bedingungen für ein Verfahren zum Identifizieren von Inhibitoren der Thymidylat-Kinase bzw. zur Bestimmung der Aktivität der erfindungsgemäßen Polypeptide zu ermitteln, kann es vorteilhaft sein, den jeweiligen K_{M}-Wert des verwendeten erfindungsgemäßen Polypeptids zu bestimmen. Dieser gibt Aufschluss über die bevorzugt zu verwendende Konzentration des bzw. der Substrate. Im Fall der Thymidylat-Kinase aus Hefe konnte ein K_{M} von 0,17 mM für dTMP und ein K_{M} von 0,16 mM für ATP bestimmt werden (Abbildung 5).

Mit Hilfe der vorstehend beispielhaft beschriebenen Verfahren konnten im Rahmen der vorliegenden Erfindung Verbindungen identifiziert werden, die die pilzliche Thymidylat-Kinase inhibieren, und die in der Lage sind, Pilze verschiedener Spezies zu schädigen (z.B. Wachstumshemmung) bzw. abzutöten.

Es versteht sich von selbst, dass neben den genannten Verfahren zur Bestimmung der enzymatischen Aktivität einer Thymidylat-Kinase bzw. der Inhibition dieser Aktivität und zum Identifizieren von Fungiziden auch andere, z.B. bereits bekannte, Verfahren bzw. Hemmtests verwendet werden können, solange diese Verfahren es gestatten, die Aktivität einer Thymidylat-Kinase zu bestimmen und eine Inhibition dieser Aktivität durch eine Testverbindung zu erkennen.

Die mit Hilfe eines erfindungsgemäßen Verfahrens identifizierten Inhibitoren einer erfindungsgemäßen Thymidylat-Kinase sind geeignet, Pilze, bevorzugt pflanzenpathogene Pilze, zu schädigen oder zu töten.

Um zu überprüfen, ob die in einem erfindungsgemäßen Verfahren identifizierten Verbindung geeignet sind, um *in vivo* zur Bekämpfung von pflanzenpathogenen Pilzen eingesetzt zu werden, kann z.B. in die Kavitäten von Mikrotiterplatten eine Lösung des zu prüfenden Wirkstoffs pipettiert werden. Nachdem das Lösungsmittel abgedampft ist, wird zu jeder Kavität Medium hinzugefügt. Das Medium wird vorher mit einer geeigneten Konzentration von Sporen bzw. Mycel des zu prüfenden Pilzes versetzt. Die resultierenden Konzentrationen des Wirkstoffes betragen z.B. 0,1, 1, 10 und 100 ppm.

Die Platten werden anschließend auf einem Schüttler bei einer Temperatur von 22°C inkubiert, bis in der unbehandelten Kontrolle ein ausreichendes Wachstum feststellbar ist.

Die vorliegende Erfindung bezieht sich daher ebenfalls auf die Verwendung von Modulatoren der Thymidylat-Kinase aus Pilzen, bevorzugt aus pflanzenpathogenen Pilzen als Fungizide.

Die vorliegende Erfindung bezieht sich gleichermaßen auf Verfahren zum Bekämpfen von pflanzenpathogenen Pilzen, indem man einen Inhibitor der pilzlichen Thymidylat-Kinase, gegebenenfalls in einer geeigneten Formulierung, auf den zubekämpfenden Pilz und/oder seinen Lebensraum einwirken lässt.

Die vorliegende Erfindung bezieht sich auch auf Fungizide, die mit Hilfe eines erfindungsgemäßen Verfahrens identifiziert wurden.

Verbindungen, die mit Hilfe eines erfindungsgemäßen Verfahrens identifiziert werden, und die aufgrund der Inhibition der pilzlichen Thymidylat-Kinase eine fungizide Wirkung aufweisen, können dann zur Herstellung von fungiziden Mitteln verwendet werden.

Die identifzierten Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Beim Einsatz der erfindungsgemäßen Verbindungen als Fungizide können die Aufwandmengen je nach Applikation innerhalb größerer Bereiche variiert werden.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die nachfolgenden Beispiele illustrieren verschiedene Aspekte der vorliegenden Erfindung und sind nicht limitieren auszulegen.

### Beispiele

### Beispiel 1

### Herstellung von cdc8 Knock-out Mutanten in U. maydis

### Kultivierung von U. maydis

Die Stämme wurden bei 28°C auf PD-, YEPS- oder geeigneten Minimalmedien (Holliday, 1974; Tsukuda et al., 1988) angezogen. Die Bildung dikaryotischer Filamente wurde nach dem Auftropfen von Stämmen auf PD-Plattenmedien, die 1% Charcoal enthielten, beobachtet (Holliday, 1974). Pathogenitätstests wurden wie beschrieben durchgeführt (Gillissen et al., 1992). Übernachtkulturen der Stämme wurden in einer Konzentration von 4x10⁷ Zellen resuspendiert und jungen Maispflanzen (Gaspe' Flint) injiziert bzw. aufgetropft. Für jeden Stamm wurden mindestens 25 Pflanzen infiziert und entstehende Tumore nach 14-21 Tagen untersucht.

### Herstellung der Knock-out Kassette

Molekularbiologische Standardmethoden wurden nach Sambrook et al., 1989 durchgeführt. Um *cdc8*-Nullmutanten herzustellen, wurde die 5' und die 3' Flanke des *cdc8*-Gens mittels PCR amplifiziert. Als Template diente geonomische DNA des Stammes UM518. Für die 5' Flanke (1314bp) wurden die Primer LB2 mit der Sequenz (5'-cacggcctgagtggcccggacggcgctgctgtcagttggg-3') und p15 mit der Sequenz (5'-gccaccatcgagtcaggaacgatgg-3') eingesetzt. Für die 3'-Flanke (1238bp) wurden die Primer RB1 (5'-gtgggccatctaggccggcggtgcacagggtgacatcgtcc-3') und p13 (5'-caccaatagcactcgacgcacgtcc-3') verwendet. Mit den Primern LB2 und RB1 wurden die Schnittstellen *Sfi* I (a) und *Sfi* I (b) eingeführt. Die Amplikons wurden mit *Sfi* I restringiert und mit dem 1884 bp großen *Sfi* I-Fragment aus dem Vektor pBS-isoliert (HygromycinB-Kassette) ligiert. Durch eine PCR mit den Primern LB1 (5'-ggtcatccagaagggcttctcgc-3') und RB2 (5'-gcttgccgtcgctggatcggagagg -3') wurde die 3869 bp große *cdc8*-Knock-out Kassette amplifiziert, die in die nachfolgende Transformation eingesetzt wurde (Kaemper and Schreier, 2003).

### Herstellung von Protoplasten von U maydis

50 ml einer Kultur in YEPS Medium wurden bei 28°C bis zu einer Zelldichte von ca. 5x10⁷/ml (OD 0.6 bis 1.0) angezogen und dann für 7 min. bei 2500g (Hereaus, 3500 rpm) in 50 ml Falkonröhrchen abzentrifugiert. Das Zellpellet wurde in 25 ml SCS-Puffer (20 mM NaCitrat pH 5.8, 1.0 M Sorbit, (20 mM NaCitrat/1.0 M Sorbit und 20 mM Zitronensäure/1.0 M Sorbit mischen und mit pH-Meter auf pH 5.8 einstellen)) resuspendiert, erneut 7 min. bei 2500 g (3500 rpm) zentrifugieren und das Pellet in 2 ml SCS-Puffer, pH 5,8, mit 2,5 mg/ml Novozym 234 resuspendiert. Die Protoplastierung erfolgte bei Raumtemperatur und wurde mikroskopisch alle 5 min. verfolgt. Die Protoplasten wurden dann mit 10 ml SCS-Puffer gemischt und bei 1100 g (2300 rpm) 10 min. zentrifugiert, der Überstand wurde verworfen. Das Pellet wurde vorsichtig in 10 ml SCS Puffer resuspendiert und erneut zentrifugiert. Der Waschvorgang mit SCS-Puffer wurde zweimal wiederholt, das Pellet wurde in 10 ml STC-Puffer gewaschen. Schließlich wurde das Pellet in 500 µl kaltem STC-Puffer (10 mM Tris/HCL pH 7.5, 1.0 M Sorbit, 100 mM CaCl₂) resuspendiert und auf Eis gehalten. Aliquots können mehrere Monate bei -80°C gelagert werden.

### Transformation von U maydis

Die Transformation eines diploiden *U. maydis* Stammes erfolgte nach Schulz et al., 1990. Die Isolierung genomischer *U. maydis* DNA erfolgte wie bei Hoffman and Winston, 1987 beschrieben bzw. nach dem Protokoll des Firma Qiagen (DNeasy-Kit).

Zur Transformation wurden max. 10 µl DNA (optimal 3-5 µg) in ein 2 ml Eppendorfröhrchen übertragen, 1 µl Heparin (15 µg/µl) (SIGMA H3125) zugegeben und dann 50 µl Protoplasten zugesetzt und 10 min. auf Eis inkubiert. 500 µl 40% (w/w) PEG3350 (SIGMA P3640) in STC (steril filtriert) wurden zugesetzt, vorsichtig mit der Protoplastensuspension gemischt und 15 min. auf Eis inkubiert. Das Ausplattieren erfolgte auf Gradienten-Platten (unterer Agar: 10 ml YEPS-1,5% Agar-1M Sorbitol mit Antibiotikum; kurz vor dem Ausplattieren wurde die untere Agarschicht mit 10 ml YEPS- 1,5% Agar-1M Sorbitol überschichtet, die Protoplasten ausgestrichen und die Platten für 3-4 Tage bei 28°C inkubiert.

Der Nachweis der homologen Rekombination in einen genomischen Lokus von *cdc8* wurde mittels Standardmethoden (PCR oder Southernanalyse) von isolierter genomischer DNA durchgeführt. Hierbei wurde gezeigt, dass die Integration der *cdc8*-Knock-out Kassette in einen genomischen *cdc8*-Lokus stattgefunden hat und somit eine Wildtypkopie des *cdc8*-Gens ersetzt wurde, während die zweite Kopie in diesem diploiden Stamm erhalten blieb. Die auf diese Weise entstandenen heterozygoten *cdc8* Mutanten wurden nachfolgend in den Pathogenitätstest eingesetzt.

### Sporenanalyse von U maydis

Aus den im Pathogenitätstest erzeugten Tumoren wurden Sporen isoliert. Im Anschluss wurden die entstehenden Sporidien vereinzelt und phäno- bzw. genotypisch untersucht. Die phänotypische Analyse erfolgte mittels Wachstumsversuchen auf geeigneten Voll- bzw. Minimalmedien (Holliday, 1974; Tsukuda et al., 1988). Dabei zeigte sich, das keine analysierten Sporidien unter selektiven Bedingungen wuchsen. Es wurden keine diploides Stämme gefunden. Dies war ein erster Hinweis darauf, dass der Phänotyp der *cdc8*-Null-Mutante lethal war. Die genotypischen Untersuchungen erfolgten durch Southern- bzw. PCR basierter Analyse der Sporidien. Hierbei wurde gefunden, dass kein lebensfähiger, haploider Stamm identifiziert werden konnte, in dem das *cdc8-*-Gen durch die *cdc8*-Knock-out-Kassette ersetzt war. Aus diesen Ergebnissen wurde abschließend geschlossen, dass der Knock-out des *cdc8-*Gens in *Ustilago maydis* zu einem lethalen Phänotyp führt.

### Beispiel 2

### Klonierung, Expression und Reinigung von cdc8 bzw. CDC8 aus Saccharomyces cerevisiae

Für die Klonierung von *cdc8* bzw. dessen Expression wurde der ORF aus genomischer DNA aus *Saccharomyces cerevisiae* über genspezifische Primer amplifiziert. Die entsprechende DNA, ein Amplikon von 651 bp Länge, wurde in den Vektor pENTR/D-TOPO (Invitrogen Corporation, Carlsbad, California, USA) kloniert und mittels spezifischer Rekombination (Gateway-Technologie Invitrogen) in den Vektor pDest17 (Invitrogen) transferiert. Das resultierende Plasmid pDest17-cdc8 enthält die vollständige kodierende Sequenz von cdc8 in einer N-terminalen Fusion mit einem His6-Tag aus dem Vektor. Das CDC8 Fusionsprotein besitzt eine berechnete Masse von 27 kDa (Abbildung 3).

Für die heterologe Expression wurde das Plasmid pDest17-cdc8 in *E. coli* BL21(DE3)pLysS transformiert. Von den Transformanten wurde eine Vorkultur in 50 ml Selektionsmedium angeimpft. Diese Zellen wurden über Nacht bei 37°C inkubiert und anschließend 1:15 in Selektionsmedium (LB-Medium mit 80 µg/ml Ampicillin) verdünnt. Die Induktion erfolgte bei einer OD₆₀₀ₙₘ von 0,7 mit 2 mM IPTG (Endkonzentration) bei 28°C. Nach 24 h Induktion wurden die Zellen geerntet und direkt aufgearbeitet.

Der Aufschluss erfolgte durch Sonifizieren in Aufschlusspuffer (20 mM Tris, 50 mM NaCl, 40 µM TMP, 10% Glycerol, 4 mM MgCl₂, pH 7,4). Die durch Zentrifugation (20 min bei 4°C, 10 000 g) erhaltene Cytoplasmafraktion wurde für die Aufreinigung des exprimierten Proteins verwendet. Die Reinigung erfolgte nach dem Standartprotokoll des Herstellers der 5 mL HiTrap Chelating (Ni) Säule (Amersham Biosciences). Dabei wurde die Säule mit 10 Säulenvolumen (SV) Aufschlusspuffer equilibriert und nach dem Probenauftrag mit 7 SV Aufschlusspuffer gewaschen. Anschließend wurde mit 10 SV Waschpuffer (20 mM Tris, 0,5 M NaCl, 4 mM MgCl₂, 10% Glycerol, 40 µM TMP, 20 mM Imidazol, pH 7,4) gewaschen und saubere Thymidylat-Kinase eluiert (20 mM Tris, 50 mM NaCl, 4 mM MgCl₂, 10% Glycerol, 10 µM TMP, 400 mM Imidazol, pH 7,4). Das gereinigte Protein wurde in Elutionspuffer bei -80°C gelagert.

### Beispiel 3

### Identifizierung von Modulatoren der Thymidylat-Kinase in 384-Well-MTP in einem gekop-pelten Assay

Zur Identifizierung von Modulatoren der Thymidylat-Kinase wurden 384-Well-Mikrotiterplatten von Greiner verwendet.

In die erste Spalte wurde die Negativ-Kontrolle pipettiert. Diese setzte sich zusammen aus 5µl 5% DMSO und 25 µl Puffer1 (75 mM MOPS-Puffer pH 7,5, 10 mM MgCl2, 5 mM DTT, 1 mM TMP, 100 mM NaCL, 50 mM KCl). In die zweite Spalte wurde Positiv-Kontrolle pipetiert, die sich aus 5µl 5% DMSO und 25µl Puffer2 (Puffer1 mit 1,6µg/ml Thymidylat-Kinase) zusammensetzt.

In die verbleibenden Spalten wurde eine Prüfsubstanz in einer Konzentration von 2 µM in DMSO vorgelegt, wobei zum Verdünnen der Substanz auf ein Volumen von 5 µl Wasser verwendet wurde. Nach Zugabe von 20 µl Puffer2 wurden zum Start der Reaktion 20 µl Puffer3 (75 mM MOPS-Puffer pH 7,5, 10 mM MgCL2, 0,75 mM NADH, 1,25 mM ATP, 1,25 mM PEP, 6,25 mM DTT, 0,025% Tween 20, 125 mM NaCL, 62,5 mM KCl, 5U/mL PK, 5U/mL LDH) in alle Wells zugegeben.

Es folgte Inkubation bei 37°C für 40 Minuten und die abschließende Messung durch Bestimmung der Absorption bei 340 nm in einem für MTP geeigneten SPECTRAFluor Plus von Tecan.

### Beispiel 4

### Nachweis der fungiziden Wirkung der identifizierten Inhibitoren der Thymidylat-Kinase

In die Kavitäten von Mikrotiterplatten werden eine methanolische Lösung des anhand eines erfindungsgemäßen Verfahrens identifizierten Wirkstoffs in der gewünschten Menge, versetzt mit einem Emulgator, pipettiert. Nachdem das Lösungsmittel abgedampft ist, werden je Kavität 200 µl Potatoe-Dextrose-Medium hinzugefügt. Das Medium wird vorher mit geeigneten Konzentrationen von Sporen bzw. Mycelen des zu prüfenden Pilzes versetzt.

Die resultierende Konzentration des Emulgators beträgt 300 ppm.

Die Platten werden anschließend auf einem Schüttler bei einer Temperatur von 22°C inkubiert, bis in der unbehandelten Kontrolle ein ausreichendes Wachstum feststellbar ist. Die Auswertung erfolgt photometrisch bei einer Wellenlänge von 620 nm. Aus den Messdaten der verschiedenen Konzentrationen wird die Wirkstoffdosis, die zu einer 50%igen Hemmung des Pilzwachstums gegenüber der unbehandelten Kontrolle führt (ED₅₀), berechnet.

### Literatur

1. Baranowska, H., Zaborowska, D., Jachymczyk, W. J., and Zuk, J. (1990). Role of the CDC8 gene in the repair of single strand breaks in DNA of the yeast Saccharomyces cerevisiae. In Current Genetics, pp. 175-179.
2. Bone, R., Cheng, Y. C., and Wolfenden, R. (1986). Inhibition of adenosine and thymidylate kinases by bisubstrate analogs. In Journal of Biological Chemistry (United States), pp. 16410-16413.
3. Davies, L. C., Stock, J. A., Barrie, S. E., Orr, R. M., and Harrap, K. R. (1988). Dinucleotide analogues as inhibitors ofthymidine kinase, thymidylate kinase, and ribonucleotide reductase. In Journal of Medicinal Chemistry (United States), pp. 1305-1308.
4. Gillissen, B., Bergemann, J., Sandmann, C., Schroeer, B., Bolker, M., and Kahmann, R. (1992). A two-component regulatory system for self/non-self recognition in Ustilago maydis. In Cell (United States), pp. 647-657.
5. Haouz, A., Vanheusden, V., Munier-Lehmann, H., Froeyen, M., Herdewijn, P., Van Calenbergh, S., and Delarue, M. (2003). Enzymatic and Structural Analysis of Inhibitors Designed against Mycobacterium tuberculosis Thymidylate Kinase. In Journal of Biological Chemistry, pp. 4963-4971.
6. Hoffman, C. S., and Winston, F. (1987). A ten-minute DNA preparation from yeast efficiently releases autonomous plasmids for transformation of Escherichia coli. In Gene, pp. 267-272.
7. Holliday, R. (1974). Ustilago maydis. In Handbook of Genetics, R. C. King, ed. (New York, Plenum), pp. 575-595.
8. Jong, A. Y. S., and Campbell, J. L. (1984). Characterization of Saccharomyces cerevisiae thymidylate kinase, the CDC8 gene product. General properties, kinetic analysis, and subcellular localization. In Journal ofBiological Chemistry, pp. 14394-14398.
9. Kaemper, J., and Schreier, P. (2003). A PCR method for the rapid construction of vectors for the generation of deletion mutants by gene knockout. In PCT Int. Appl. (WO, (Bayer Aktiengesellschaft, Germany).), pp. 51 pp.
10. Lavie, A., Konrad, M., Brundiers, R., Goody, R. S., Schlichting, I., and Reinstein, J. (1998). Crystal structure of yeast thymidylate kinase complexed with the bisubstrate inhibitor P1-(5'-adenosyl) P5-(5'-thymidyl) pentaphosphate (TP5A) at 2.0 A resolution: implications for catalysis and AZT activation. In Biochemistry (United States), pp. 3677-3686.
11. Lavie, A., Vetter, I. R., Konrad, M., Goody, R. S., Reinstein, J., and Schlichting, I. (1997). Structure of thymidylate kinase reveals the cause behind the limiting step in AZT activation. In Nature Structural Biology, pp. 601-604.
12. Manallack, D. T., Pitt, W. R., Herdewijn, P., Balzarini, J., De Clercq, E., Sanderson, M. R., Sohi, M., Wien, F., Munier-Lehmann, H., Haouz, A., and Delarue, M. (2002). Database searching for thymidine and thymidylate kinase inhibitors using three-dimensional structure-based methods. In Journal of Enzyme Inhibition and Medicinal Chemistry, pp. 167-174.
13. Newlon, C. S., and Fangman, W. L. (1975). Mitochondrial DNA synthesis in cell cycle mutants of Saccharomyces cerevisiae. In Cell (Cambridge, MA, United States), pp. 423-428.
14. Ostermann, N., Schlichting, I., Brundiers, R., Konrad, M., Reinstein, J., Veit, T., Goody, R. S., and Lavie, A. (2000). Insights into the phosphoryltransfer mechanism of human thymidylate kinase gained from crystal structures of enzyme complexes along the reaction coordinate. In Structure (London), pp. 629-642.
15. Ostermann, N., Segura-Pena, D., Meier, C., Veit, T., Monnerjahn, C., Konrad, M., and Lavie, A. (2003). Structures of human thymidylate kinase in complex with prodrugs: implications for the structure-based design of novel compounds. In Biochemistry (United States), pp. 2568-2577.
16. Rossi, M., and Wodward, D. O. (1975). Enzymes of deoxythymidine triphosphate biosynthesis in Neurospora crassa mitochondria. In Journal of Bacteriology, pp. 640-647.
17. Sambrook, J., Fritsch, E. F., and Maniatis, T. (1989). Molecular cloning: A laboratory manual. (Cold spring harbor, New York, Cold spring harbor laboratory press).
18. Schild, D., and Byers, B. (1978). Meiotic effects of DNA-defective cell division cycle mutations of Saccharomyces cerevisiae. In Chromosoma, pp. 109-130.
19. Schulz, B., Banuett, F., Dahl, M., Schlesinger, R., Schaefer, W., Martin, T., Herskowitz, I., and Kahmann, R. (1990). The b alleles of U. maydis, whose combinations program pathogenic development, code for polypeptides containing a homeodomain-related motif. In Cell (Cambridge, MA, United States), pp. 295-306.
20. Sclafani, R. A., and Fangman, W. L. (1984). Yeast gene CDC8 encodes thymidylate kinase and is complemented by herpes thymidine kinase gene TK. In Proceedings of the National Academy of Sciences of the United States of America, pp. 5821-5825.
21. Tsukuda, T., Carleton, S., Fotheringham, S., and Holloman, W. K. (1988). Isolation and characterization of an autonomously replicating sequence from Ustilago maydis. In Molecular and Cellular Biology, pp. 3703-3709.
22. Zamb, T. J., and Roth, R. (1977). Role of mitotic replication genes in chromosome duplication during meiosis. In Proceedings of the National Academy of Sciences of the United States of America, pp. 3951-3955.

## Patentansprüche

1. Verfahren zum Identifizieren von Fungiziden, **dadurch gekennzeichnet, dass** man
(a) eine Wirtszelle, die eine Thymidylat-Kinase in ausreichender Menge exprimiert oder ein Polypeptid mit der enzymatischen Aktivität einer Thymidylat-Kinase mit einer chemischen Verbindung oder einem Gemisch von chemischen Verbindungen unter Bedingungen, die die Interaktion der chemischen Verbindung mit dem Polypeptid erlauben, in Kontakt bringt,
(b) die enzymatische Aktivität der Thymidylat-Kinase bei Abwesenheit einer chemischen Verbindung mit der enzymatischen Aktivität der Thymidylat-Kinase bei Anwesenheit einer chemischen Verbindung oder eines Gemisches von chemischen Verbindungen vergleicht, und
(c) die chemische Verbindung, die die Thymidylat-Kinase spezifisch inhibiert, bestimmt.

2. Verfahren gemäß Anspruch 1, **dadurch gekennzeichnet, dass** man
(a) das bei der Reaktion der Thymidylat-Kinase entstehende ADP mit Hilfe einer Pyruvatkinase zu ATP umsetzt,
(b) das dabei entstehende Pyruvat mit Hilfe einer Lactatdehydrogenase unter NADH-Verbrauch zu Lactat umsetzt, und
(c) den Verbrauch des NADHs verfolgt.

3. Verfahren gemäß Anspruch 2, **dadurch gekennzeichnet, dass** man eine Inhibition der enzymatischen Aktivität anhand einer geringeren Zunahme der ADP-Konzentration bestimmt.

4. Verfahren gemäß einem der Ansprüche 1 bis 3, **dadurch gekennzeichnet, dass** man in einem weiteren Schritt die fungizide Wirkung der identifizierten Verbindung testet, indem man sie mit einem Pilz in Kontakt bringt.

5. Verfahren gemäß einem der Ansprüche 1 bis 4, **dadurch gekennzeichnet, dass** man eine Thymidylat-Kinase aus *S. cerevisiae* verwendet.

6. Verfahren gemäß einem der Ansprüche 1 bis 4, **dadurch gekennzeichnet, dass** man eine Thymidylat-Kinase aus einem pflanzenpathogenen Pilz verwendet.

7. Verwendung von Polypeptiden mit der Aktivität einer Thymidylat-Kinase zum Identifizieren von Fungiziden.

8. Verwendung von Inhibitoren pilzlicher Polypeptide mit der Aktivität einer Thymidylat-Kinase als Fungizide.

9. Verwendung von Inhibitoren eines Polypeptids mit der Aktivität einer Thymidylat-Kinase, welche durch ein Verfahren gemäß einem der Ansprüche 1 bis 4 identifiziert werden, als Fungizide.

10. Verfahren zum Bekämpfen pflanzenpathogener Pilze, **dadurch gekennzeichnet, dass** man Inhibitoren pilzlicher Polypeptide mit der Aktivität einer Thymidylat-Kinase auf die pflanzenpathogenen Pilze und/oder ihren Lebensraum einwirken lässt.
